# EUROPÄISCHE PATENTSCHRIFT

(11) **EP 4 072 471 B1**
(45) Veröffentlichungstag und Bekanntmachung des Hinweises auf die Patenterteilung: **06.03.2024**
(21) Anmeldenummer: 20833738.6
(22) Anmeldetag: 10.12.2020
(51) Int. Cl.: A61F 2/16

(54) **KARTUSCHE UND INJEKTOR MIT DER KARTUSCHE**
CARTRIDGE, AND INJECTOR COMPRISING THE CARTRIDGE
CARTOUCHE ET INJECTEUR ÉQUIPÉ DE CETTE CARTOUCHE

(30) Priorität: 12.12.2019 DE 102019134183
(43) Veröffentlichungstag der Anmeldung: 19.10.2022
(73) Patentinhaber: Carl Zeiss Meditec AG, 07745 Jena (DE)
(72) Erfinder: DAMM, Niklas, 10409 Berlin (DE)
(74) Vertreter: PATERIS Patentanwälte PartmbB
(86) Internationale Anmeldenummer: PCT/EP2020/085601
(87) Internationale Veröffentlichungsnummer: WO 2021/116321

(56) Entgegenhaltungen:
- EP-A2- 0 937 443
- WO-A1-03/045285
- DE-A1-102005 004 598

## Beschreibung

Die Erfindung betrifft eine Kartusche und einen Injektor mit der Kartusche.

Bei der Kataraktbehandlung eines Auges wird herkömmlich ein Schnitt in die Hornhaut des Auges eingebracht, der so groß ist, dass eine Kanüle durch den Schnitt in das Auge eingeführt werden kann. Nachdem der Schnitt in die Hornhaut eingebracht wurde, wird die Linse des Auges mittels Phakoemulsifikation zerkleinert und anschließend aus dem Kapselsack des Auges abgesaugt. Danach wird eine Intraokularlinse mittels eines Injektors in den Kapselsack eingesetzt. Dazu weist der Injektor einen Kolben auf, der eingerichtet ist, die Intraokularlinse durch eine Injektorspitze aus dem Injektor herauszuschieben. Es existieren Injektoren, bei denen die Intraokularlinse erst kurz vor der Kataraktbehandlung in den Injektor eingesetzt wird. Dies erfolgt beispielsweise, indem eine Kartusche, in der die Intraokularlinse sich befindet, in den Injektor eingesetzt wird. Es ist dabei nachteilig, dass es sich bei der Kartusche und dem Injektor um Bauteile handelt, die einen aufwändigen und komplizierten Aufbau haben.

DE 10 2005 004 598 A1 offenbart eine Vorrichtung zum Implantieren einer Intraokularlinse.

Aufgabe der Erfindung ist es daher, einen einfachen Aufbau für eine Kartusche und einen Injektor mit einer solchen Kartusche zu schaffen. Die Erfindung ist im beigefügten Anspruchssatz beschrieben.

Die erfindungsgemäße Kartusche weist eine erste Öffnung, eine zweite Öffnung, einen Kanal, der sich von der ersten Öffnung bis zu der zweiten Öffnung erstreckt und eine Verjüngung aufweist, eine Intraokularlinse, die in dem Kanal zwischen der ersten Öffnung und der zweiten Öffnung angeordnet ist, und einen Kartuschenkolben auf, der in dem Kanal zwischen der Intraokularlinse und der zweiten Öffnung angeordnet ist und eingerichtet ist, durch ein Verschieben des Kartuschenkolbens zu der ersten Öffnung hin die Intraokularlinse zuerst vorzuspannen, danach durch ein Verschieben der Intraokularlinse in der Verjüngung zu falten und anschließend via die erste Öffnung aus dem Kanal zu drücken, wobei die Kartusche eingerichtet ist, mit einer Spritze gekuppelt zu werden, so dass mittels der Spritze via die zweite Öffnung ein Fluid in den Kanal eindrückbar ist und dadurch der Kartuschenkolben in einer Einschieberichtung zu der ersten Öffnung hin antreibbar ist, wobei der Kartuschenkolben ein erstes Kartuschenkolbenlängsende, ein zweites Kartuschenkolbenlängsende und eine Fluidleitung aufweist, die sich von dem ersten Kartuschenkolbenlängsende bis zu dem zweiten Kartuschenkolbenlängsende erstreckt und via die das Fluid von dem zweiten Kartuschenkolbenlängsende bis zu dem ersten Kartuschenkolbenlängsende und anschließend bis zu der Intraokularlinse eindrückbar ist.

Indem die Kartusche mit der Spritze gekuppelt wird, wird ein Injektor hergestellt, mit dem die Intraokularlinse in einen Kapselsack eines Auges eingesetzt werden kann. Sowohl bei der Kartusche, die den Kanal mit der Verjüngung und den Kartuschenkolben aufweist, als auch bei der Spritze handelt es sich um Bauteile mit einem einfachen Aufbau. Damit ist der Injektor einfach und kostengünstig herstellbar. Durch das Vorsehen der Fluidleitung ist es möglich, die Intraokularlinse mit dem Fluid zu benetzen, bevor die Intraokularlinse in den Kapselsack des Auges eingesetzt wird. Dadurch kann eine Reibung der Intraokularlinse in dem Kanal verringert werden. Durch ein Wählen der Form der Fluidleitung kann zudem eingestellt werden, wie stark der Kartuschenkolben von dem Fluid angetrieben wird. Hat die Fluidleitung einen kleinen Querschnitt, so wird der Kartuschenkolben eher stark angetrieben und eine eher größere Menge des Fluids gelangt bis zu der Intraokularlinse. Hat die Fluidleitung einen großen Querschnitt, so wird der Kartuschenkolben eher schwach angetrieben und eine eher kleine Menge des Fluids gelangt bis zu der Intraokularlinse.

Es ist bevorzugt, dass das erste Kartuschenkolbenlängsende projiziert in der Einschieberichtung einen größeren Querschnitt hat als das zweite Kartuschenkolbenlängsende projiziert in der Einschieberichtung. Besonders bevorzugt ist, dass der Kartuschenkolben im Bereich des ersten Kartuschenkolbenlängsendes den Kanal abdichtet, so dass kein Fluid den Kartuschenkolben an seinem äußeren Umfang passieren kann. Dadurch wird erreicht, dass die Intraokularlinse, insbesondere eine Haptik der Intraokularlinse, nicht zwischen dem Kartuschenkolben und einer Wand des Kanals eingeklemmt werden kann. Zudem gelangt das Fluid nur via die Fluidleitung bis zu der Intraokularlinse.

Der Kanal weist bevorzugt einen ersten Kanalabschnitt, in dem das zweite Kartuschenkolbenlängsende angeordnet ist, bevor das Fluid in den Kanal eingedrückt ist, und der einen ersten Querschnitt aufweist, dessen Normale parallel zu der Einschieberichtung angeordnet ist und im Wesentlichen dem in der Einschieberichtung projizierten zweiten Kartuschenkolbenlängsende entspricht, und einen zweiten Kanalabschnitt auf, der zwischen dem ersten Kanalabschnitt und der ersten Öffnung angeordnet ist und einen zweiten Querschnitt aufweist, dessen Normale parallel zu der Einschieberichtung angeordnet ist und der größer als der erste Querschnitt ist, wobei der Abstand von der ersten Öffnung zu dem ersten Kanalabschnitt länger ist als die Erstreckung des Kartuschenkolbens in der Einschieberichtung. Dadurch wird erreicht, dass am Anfang des Verschiebens des Kartuschenkolbens hin zu der ersten Öffnung das Fluid nur an das zweite Kartuschenkolbenlängsende angreift. Verschiebt der Kartuschenkolben weiter zu der ersten Öffnung hin, gelangt das zweite Kartuschenkolbenlängsende aus dem ersten Kanalabschnitt. Dadurch kann das Fluid das Längsende passieren und greift eine größere Fläche als zu dem Anfang des Verschiebens an. Unter der Annahme, dass der Druck des Fluids während des gesamten Verschiebens gleich bleibt, wirkt eine höhere Kraft auf den Kartuschenkolben, nachdem das zweite Kartuschenkolbenlängsende den ersten Kanalabschnitt verlassen hat. Weil der Abstand von der ersten Öffnung zu dem ersten Kanalabschnitt länger ist als die Erstreckung des Kartuschenkolbens, geschieht dies, solange der Kartuschenkolben noch vollständig innerhalb des Kanals angeordnet ist. Indem sich die Kraft während des Verschiebens des Kartuschenkolbens erhöht, kann dadurch vorteilhaft kompensiert werden, dass die Intraokularlinse bei ihrem Verschieben zu der ersten Öffnung hin immer weiter gequetscht wird und somit eine immer höhere Widerstandskraft erzeugt.

Es ist bevorzugt, dass der Kartuschenkolben einen ersten Abschnitt, der das erste Kartuschenkolbenlängsende aufweist und flexibel ist, einen zweiten Abschnitt, der das zweite Kartuschenkolbenlängsende aufweist, und einen dritten Abschnitt aufweist, der zwischen dem ersten Abschnitt und dem zweiten Abschnitt angeordnet ist und eine Aussparung aufweist, die eingerichtet ist, Teile des ersten Abschnitts aufzunehmen, wenn der Kartuschenkolben in die Verjüngung gelangt. Dadurch kann sich der erste Abschnitt in Richtung zu dem zweiten Kartuschenkolbenlängsende hin verbiegen, wenn der Kartuschenkolben in die Verjüngung verschiebt, und in platzsparender Weisung in die Aussparung gelangen. Dabei ist besonders bevorzugt, dass der erste Abschnitt an seiner der Intraokularlinse abgewandten Seite eine Nut aufweist. Dadurch kann sich der erste Abschnitt leichter in Richtung zu dem zweiten Kartuschenkolbenlängsende hin verbiegen.

Die Fluidleitung weist bevorzugt eine Kurve auf. Dadurch erhöht sich der Strömungswiderstand in der Fluidleitung, wodurch weniger des Fluids bis zu der Intraokularlinse gelangt und der Kartuschenkolben stärker angetrieben wird. Beispielsweise kann die Kurve Teil eines mäandernden Abschnitts oder eines helixförmigen Abschnitts der Fluidleitung sein.

Die Intraokularlinse weist bevorzugt einen Optikkörper auf, der vollständig zwischen der Verjüngung und dem Kartuschenkolben angeordnet ist. Dadurch ist der Optikkörper außerhalb der Verjüngung angeordnet und befindet sich in einem entspannten Zustand, bevor die Kartusche mit der Spritze gekuppelt wird. Deshalb können Kriecheffekte in dem Optikkörper während einer Lagerung der Intraokularlinse vermieden werden. Indem die Kartusche erst kurz vor einer Kataraktbehandlung mit der Spritze gekuppelt wird und erst dann der Optikkörper in einen vorgespannten Zustand gebracht werden kann, treten keine Kriecheffekte auf. Besonders bevorzugt weist die Intraokularlinse eine Haptik auf, die so in dem Kanal angeordnet ist, dass sowohl der Optikkörper als auch die Haptik sich in einem entspannten Zustand befinden. Dadurch treten auch keine Kriecheffekt in der Haptik auf.

Es ist bevorzugt, dass der Kanal gerade ist. Dadurch weist der Kanal keine Umlenkungen oder Kurven auf und weder die Kanüle, noch der Kartuschenkolben, noch die Intraokularlinse müssen um eine Umlenkung oder eine Kurve geführt werden.

Die Kartusche weist bevorzugt eine Spitze auf, in der die erste Öffnung angeordnet ist und die eingerichtet ist, in den Kapselsack eines Auges eingeführt zu werden.

Es ist bevorzugt, dass die Kartusche einen Kartuschenadapter aufweist, der eingerichtet ist, mit einem Spritzenadapter der Spritze in Eingriff zu stehen, um die Kartusche mit der Spritze zu kuppeln. Durch das Vorsehen des Kartuschenadapters kann die Kartusche besonders einfach mit der Spritze gekuppelt werden.

Der Kartuschenadapter ist bevorzugt ein Steckeradapter einer Luer-Verbindung oder ein Buchsenadapter einer Luer-Verbindung. Alternativ ist der Kartuschenadapter bevorzugt ein Teil eines Klemmverschlusses, eines Schnappverschlusses, eines Bajonettverschluss oder eines Schraubverschlusses. In dem Fall, dass der Kartuschenadapter Teil eines Schraubverschlusses ist, weist der Kartuschenadapter ein Außengewinde oder ein Innengewinde auf.

Der erfindungsgemäße Injektor weist die Kartusche und die Spritze auf, wobei die Kartusche mit der Spritze gekuppelt ist, die Spritze das Fluid aufweist und der Injektor eingerichtet ist, durch ein Eindrücken des Fluids in den Kanal via die zweite Öffnung mittels der Spritze den Kartuschenkolben in der Einschieberichtung zu der ersten Öffnung hin zu verschieben.

Die Spritze weist bevorzugt eine Kanüle auf, durch die das Fluid aus der Spritze herausdrückbar ist. Dadurch kann die Spritze, bevor die Kartusche mit der Spritze gekuppelt wird, für andere Aufgaben eingesetzt werden. Beispielsweise kann mit der Spritze das Fluid in den Kanal eingebracht werden, um die Kartusche und/oder die Intraokularlinse zu schmieren, oder das Fluid kann in den Kapselack eingebracht werden.

Es ist bevorzugt, dass die Kanüle so lang ist, dass, wenn die Kartusche mit der Spritze gekuppelt wird, die Kanüle den Kartuschenkolben so weit in Richtung zu der ersten Öffnung hin verschiebt, dass dabei die Intraokularlinse vorgespannt wird. Es ist auch denkbar, dass dabei die Intraokularlinse gefaltet wird. Indem die Intraokularlinse vorgespannt und optional gefaltet wird, wird eine Abdichtung des Kanals bewirkt.

Der Kartuschenkolben weist bevorzugt an seinem der Spritze zugewandten Ende eine Kartuschenkolbenaussparung auf, die eingerichtet ist, das dem Kartuschenkolben zugewandte Längsende der Kanüle aufzunehmen, wenn die Kartusche mit der Spritze gekuppelt wird. Durch die Kartuschenkolbenaussparung hat der Kartuschenkolben eine definierte Stelle, an der die Kanüle den Kartuschenkolben kontaktiert.

Es ist bevorzugt, dass die Kartusche einen Kartuschenadapter aufweist und die Spritze einen Spritzenadapter aufweist, durch den die Kanüle sich erstreckt, wobei der Kartuschenadapter und der Spritzenadapter miteinander in Eingriff stehen, wodurch die Kartusche mit der Spritze gekuppelt ist und der Injektor im Bereich des Kartuschenadapters und des Spritzenadapters abgedichtet ist. Dadurch kann vorteilhaft vermieden werden, dass das Fluid, wenn es aus der Spritze herausgedrückt wird, via die zweite Öffnung aus dem Injektor gelangt.

Der Kartuschenadapter und der Spritzenadapter stehen bevorzugt formschlüssig miteinander in Eingriff. Dadurch kann mit einer hohen Genauigkeit bestimmt werden, wie weit die Kanüle in den Kanal eindringt, wenn die Kartusche mit der Spritze gekuppelt wird. Damit kann auch mit einer hohen Genauigkeit bestimmt werden, wie weit die Kanüle den Kartuschenkolben in Richtung zu der ersten Öffnung hin verschiebt und wie weit dadurch der Kartuschenkolben die Intraokularlinse in die Verjüngung schiebt.

Es ist bevorzugt, dass der Kartuschenadapter und der Spritzenadapter eine Luer-Verbindung, einen Klemmverschluss, einen Schnappverschluss, einen Bajonettverschluss oder einen Schraubverschluss bilden. In dem Fall, dass der Kartuschenadapter Teil eines Schraubverschlusses ist, weist der Kartuschenadapter ein Außengewinde oder ein Innengewinde auf.

Die Spritze weist bevorzugt einen Zylinder und einen Spritzenkolben auf, der längsverschiebbar in dem Zylinder gelagert ist, wobei der Zylinder und der Spritzenkolben einen Zylinderinnenraum begrenzen, in dem das Fluid angeordnet ist und der fluidleitend mit der Kanüle verbunden ist. Dadurch hat die Spritze einen besonders einfachen Aufbau.

Das Fluid weist bevorzugt ein Gleitmittel und/oder eine physiologische Salzlösung auf oder besteht aus dem Gleitmittel und/oder der physiologischen Salzlösung. Bei dem Gleitmittel kann es sich um ein sogenanntes opthalmisches Viskoelastikum handeln (engl.: opthalmic viscoelastic device (OVD)).

Ein Verfahren zum Zusammenbauen des Injektors weist die Schritte auf: a) Bereitstellen der Kartusche; b) Bereitstellen der Spritze, die das Fluid aufweist; c) Kuppeln der Kartusche mit der Spritze, so dass der Injektor hergestellt wird und so dass mittels der Spritze via die zweite Öffnung das Fluid in den Kanal eindrückbar ist und dadurch der Kartuschenkolben in Richtung zu der ersten Öffnung hin antreibbar ist.

Ein Verfahren zum Betreiben des Injektors weist die Schritte auf: a) Bereitstellen der Kartusche; b) Bereitstellen der Spritze, die das Fluid aufweist; c) Kuppeln der Kartusche mit der Spritze, so dass der Injektor hergestellt wird; d) Drücken der Spritze, wodurch das Fluid via die zweite Öffnung in den Kanal und via die Fluidleitung bis zu der Intraokularlinse gelangt und den Kartuschenkolben in Richtung zu der ersten Öffnung hin verschiebt, wodurch der Kartuschenkolben die Intraokularlinse zuerst vorspannt, danach durch ein Verschieben der Intraokularlinse in der Verjüngung faltet und anschließend via die erste Öffnung aus dem Kanal drückt.

Im Folgenden wird anhand der beigefügten schematischen Zeichnungen die Erfindung näher erläutert.
Figur 1 zeigt eine Kartusche.
Figur 2 zeigt eine Spritze mit einem Spritzenkolben in einer ersten Position.
Figur 3 zeigt einen Injektor, wobei die Kartusche mit der Spritze gekuppelt ist und der Spritzenkolben sich in einer zweiten Position befindet.
Figur 4 zeigt eine perspektivische Ansicht der Kartusche mit einem Teil der Spritze.
Figur 5 zeigt die perspektivische Ansicht gemäß Figur 4, jedoch mit weniger Bauteilen.
Figur 6 zeigt eine Kanüle der Spritze zusammen mit einem Teil eines Kartuschenkolbens der Kartusche.
Figuren 7 bis 16 zeigen verschiedene Ausführungsformen des Kartuschenkolbens.
Figur 17 zeigt die Kartusche und die Spritze vor und nach deren Zusammenbau zum Injektor und den Injektor in verschiedenen Zuständen seines Betriebes.

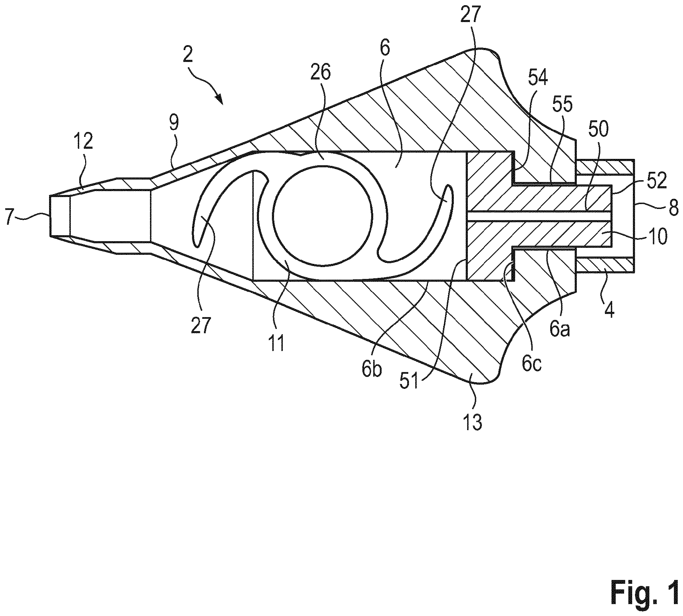

Wie es aus Figuren 1, 3 und 4 ersichtlich ist, weist eine Kartusche 2 eine erste Öffnung 7, eine zweite Öffnung 8, einen Kanal 6, der sich von der ersten Öffnung 7 bis zu der zweiten Öffnung 8 erstreckt und eine Verjüngung 9 aufweist, eine Intraokularlinse 11 und einen Kartuschenkolben 10 auf. Die Intraokularlinse 11 ist in dem Kanal 6 zwischen der ersten Öffnung 7 und der zweiten Öffnung 8 angeordnet. Der Kartuschenkolben 10 ist in dem Kanal 6 zwischen der Intraokularlinse 11 und der zweiten Öffnung 8 angeordnet und eingerichtet, durch ein Verschieben des Kartuschenkolbens 10 zu der ersten Öffnung 7 hin die Intraokularlinse 11 zuerst vorzuspannen, danach durch ein Verschieben der Intraokularlinse 11 in der Verjüngung 9 zu falten und anschließend via die erste Öffnung 7 aus dem Kanal 6 zu drücken. Der Kanal 6 ist entlang seines gesamten Umfangs geschlossen oder an mindestens einer Umfangsseite mindestens teilweise offen. Die Kartusche 2 ist eingerichtet, mit einer Spritze 3 gekuppelt zu werden, so dass mittels der Spritze 3 via die zweite Öffnung 8 ein Fluid 30 in den Kanal 6 eindrückbar ist und dadurch der Kartuschenkolben 10 in einer Einschieberichtung 22 zu der ersten Öffnung 7 hin antreibbar ist. Damit ist der Kanal 6 eingerichtet, das Fluid 30 von der Spritze 3 zu dem Kartuschenkolben 10 passieren zu lassen, so dass mit zunehmender Befüllung des Kanals 6 mit dem Fluid 30 der Kartuschenkolben 10 in Richtung zu der ersten Öffnung 7 hin antreibbar ist. Der Kartuschenkolben 10 weist ein erstes Kartuschenkolbenlängsende 51, ein zweites Kartuschenkolbenlängsende 52 und eine Fluidleitung 50 auf, die sich von dem ersten Kartuschenkolbenlängsende 51 bis zu dem zweiten Kartuschenkolbenlängsende 52 erstreckt und mittels welcher das Fluid 30 von dem zweiten Kartuschenkolbenlängsende 52 bis zu dem ersten Kartuschenkolbenlängsende 51 und anschließend bis zu der Intraokularlinse 11 eindrückbar ist, vergleiche Figuren 1, 3, 5 und 7. Damit ist der Kartuschenkolben 10 eingerichtet, das Fluid 30 von der der Intraokularlinse 11 abgewandten Seite des Kartuschenkolbens 10 bis zu der der Intraokularlinse 11 zugewandten Seite des Kartuschenkolbens 10 passieren zu lassen. An der der Intraokularlinse 11 zugewandten Seite des Kartuschenkolbens 10 benetzt das Fluid 30 die Intraokularlinse 11 und verringert dadurch deren Reibung.

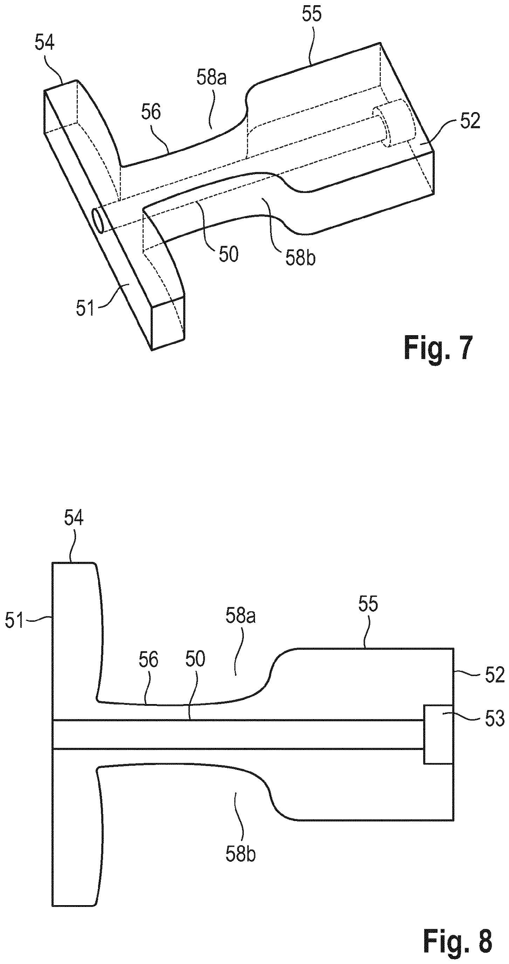

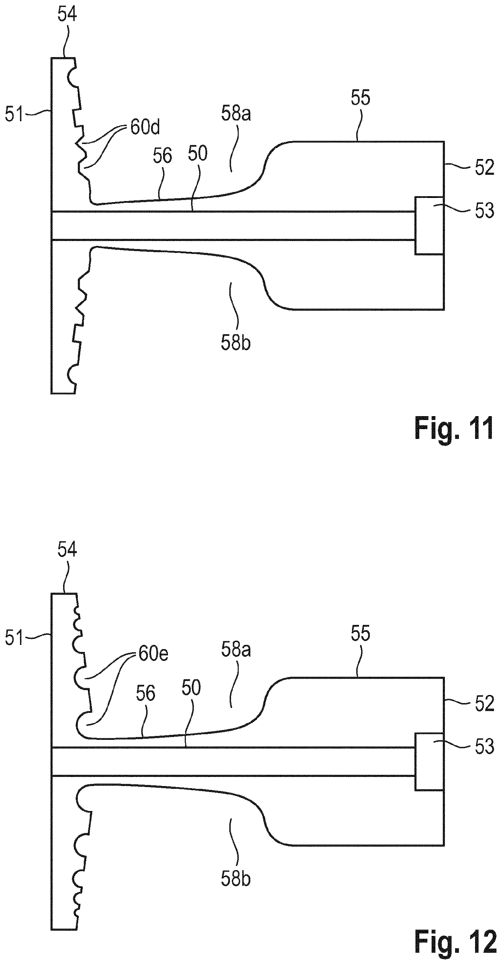

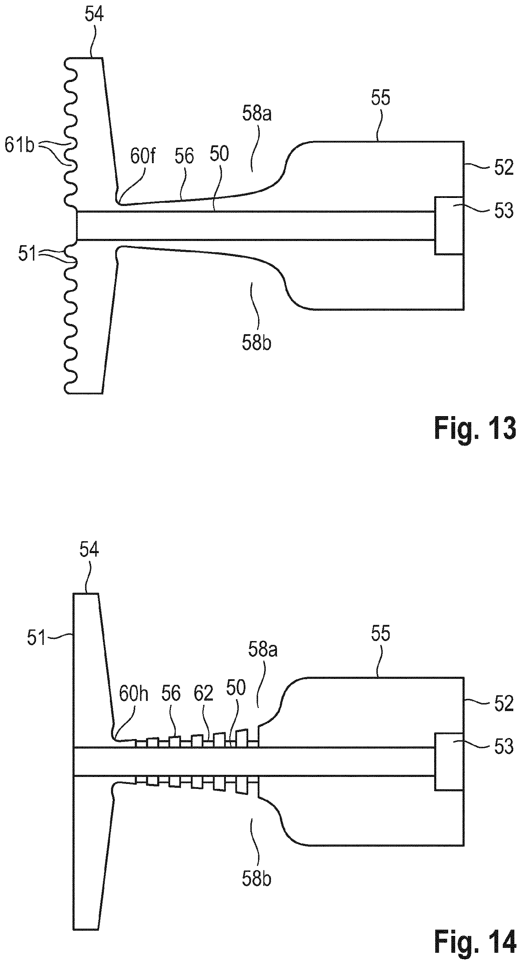

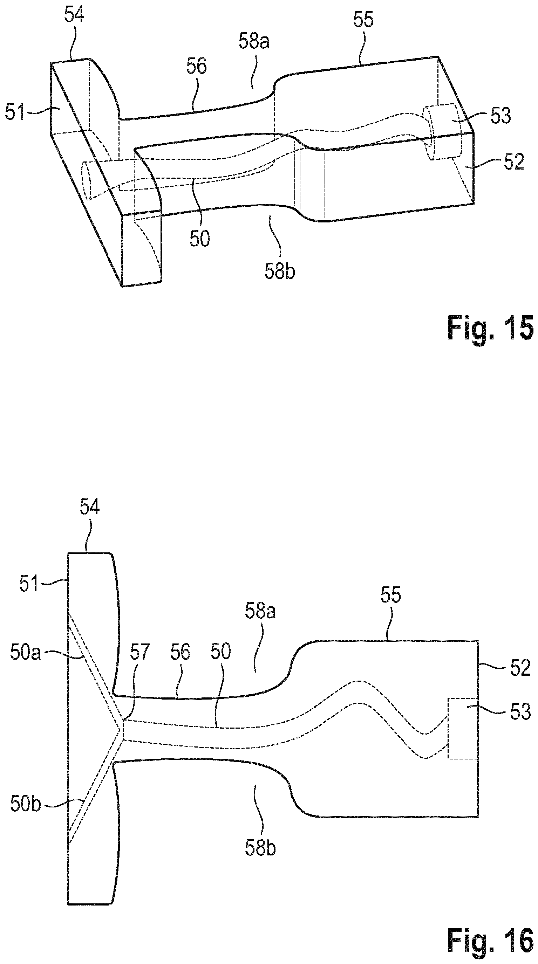

Wie es aus Figuren 1, 3 bis 5 und 7 bis 17 ersichtlich ist, kann das erste Kartuschenkolbenlängsende 51 projiziert in der Einschieberichtung 22 einen größeren Querschnitt haben als das zweite Kartuschenkolbenlängsende 52 projiziert in der Einschieberichtung 22. Der Anfang und das Ende der Fluidleitung 50 gehören dabei zu dem zweiten Kartuschenkolbenlängsende 52 beziehungsweise zu dem ersten Kartuschenkolbenlängsende 51. Weist das erste Kartuschenkolbenlängsende 51 Erhebungen und/oder Vertiefungen auf, wie es beispielsweise in Figur 13 gezeigt ist, so gehört die gesamte der Intraokularlinse 11 zugewandte Fläche des Kartuschenkolbens 10 zu dem ersten Kartuschenkolbenlängsende 51. Weist das zweite Kartuschenkolbenlängsende 52 Erhebungen und/oder Vertiefungen auf, wie es beispielsweise in Figuren 7 bis 16 gezeigt ist, so gehören die Erhebungen und die Vertiefungen zu dem zweiten Kartuschenkolbenlängsende 52.

Es ist denkbar, dass der Kartuschenkolben 10 im Bereich des ersten Kartuschenkolbenlängsendes 51 den Kanal 6 abdichtet, so dass kein Fluid 30 den Kartuschenkolben 10 an seinem äußeren Umfang passieren kann. Dadurch wird erreicht, dass die Intraokularlinse 11, insbesondere eine Haptik 27 der Intraokularlinse 11, nicht zwischen dem Kartuschenkolben 10 und einer Wand des Kanals 6 eingeklemmt werden kann. Zudem gelangt das Fluid 30 nur via die Fluidleitung 50 bis zu der Intraokularlinse 11.

Figuren 1, 3 und 17 zeigen, dass der Kanal 6 einen ersten Kanalabschnitt 6a, in dem das zweite Kartuschenkolbenlängsende 52 angeordnet ist, bevor das Fluid 30 in den Kanal 6 eingedrückt ist, und der einen ersten Querschnitt aufweist, dessen Normale parallel zu der Einschieberichtung 22 angeordnet ist und im Wesentlichen dem in der Einschieberichtung 22 projizierten zweiten Kartuschenkolbenlängsende 52 entspricht, und einen zweiten Kanalabschnitt 6b aufweisen kann, der zwischen dem ersten Kanalabschnitt 6a und der ersten Öffnung 7 angeordnet ist und einen zweiten Querschnitt aufweist, dessen Normale parallel zu der Einschieberichtung 22 angeordnet ist und der größer als der erste Querschnitt ist, wobei der Abstand von der ersten Öffnung 7 zu dem ersten Kanalabschnitt 6a länger ist als die Erstreckung des Kartuschenkolbens 10 in der Einschieberichtung 22. Beispielsweise kann ein Übergang von dem ersten Kanalabschnitt 6a zu dem zweiten Kanalabschnitt 6b mittels einer Stufe 6c verwirklicht sein. Alternativ ist denkbar, dass der Übergang mittels eines Diffusors bewerkstelligt ist.

Figuren 7 bis 16 zeigen, dass der Kartuschenkolben 10 einen ersten Abschnitt 54, der das erste Kartuschenkolbenlängsende 51 aufweist und flexibel ist, einen zweiten Abschnitt 55, der das zweite Kartuschenkolbenlängsende 52 aufweist, und einen dritten Abschnitt 56 aufweisen kann, der zwischen dem ersten Abschnitt 54 und dem zweiten Abschnitt 55 angeordnet ist und eine Aussparung 58a aufweist, die eingerichtet ist, Teile des ersten Abschnitts 54 aufzunehmen, wenn der Kartuschenkolben 10 in die Verjüngung 9 gelangt. Zudem kann der dritte Abschnitt 56 eine weitere Aussparung 58b aufweisen, die eingerichtet ist, andere Teile des ersten Abschnitts 54 aufzunehmen, wenn der Kartuschenkolben 10 in die Verjüngung 9 gelangt. Der Kartuschenkolben 10 hat somit im Wesentlichen eine T-Form, bevor er in die Verjüngung 9 gelangt, und im Wesentlichen die Form eines Stiftes, wenn der Kartuschenkolben 10 bis zu der ersten Öffnung 7 verschoben ist. Der Stift kann beispielsweise einen rechteckigen, einen kreisförmigen oder einen ovalen Querschnitt haben. Der Kartuschenkolben 10, insbesondere der erste Abschnitt 54 des Kartuschenkolbens 10, kann Silikon aufweisen oder daraus bestehen.

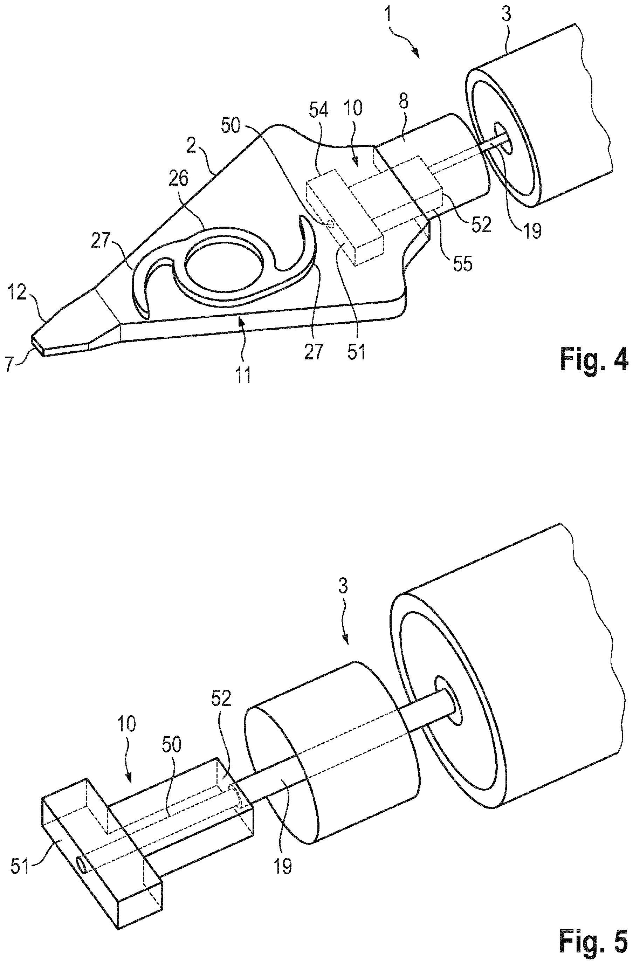

Figuren 4, 5 und 7 zeigen, dass, bevor der Kartuschenkolben 10 in die Verjüngung gelangt, das erste Kartuschenkolbenlängsende 51 und das zweite Kartuschenkolbenlängsende 52 eine rechteckige Form haben können. Ebenso ist auch denkbar, dass, bevor der Kartuschenkolben 10 in die Verjüngung gelangt, das erste Kartuschenkolbenlängsende 51 und das zweite Kartuschenkolbenlängsende 52 eine kreisförmige oder eine ovale Form haben.

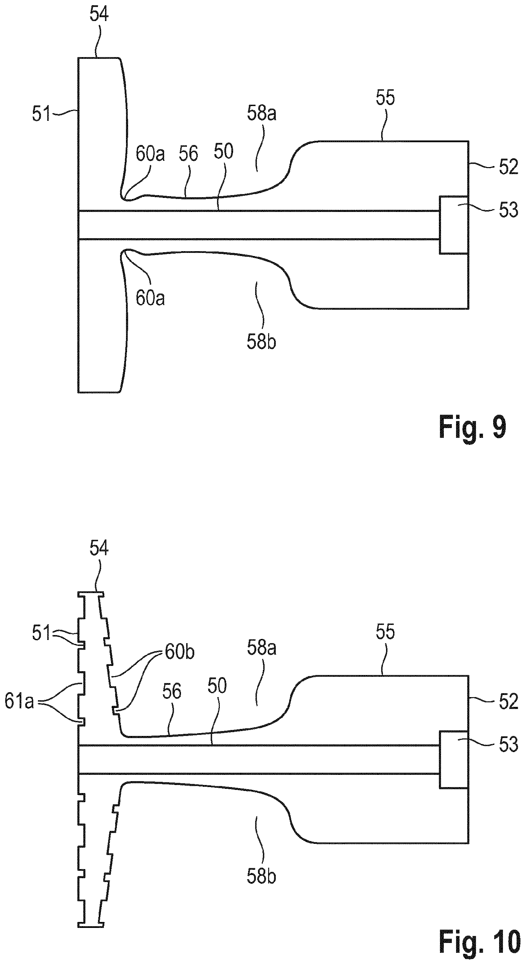

Der erste Abschnitt 54 kann an seiner der Intraokularlinse 11 abgewandten Seite eine Nut 60 aufweisen, vergleiche Figuren 9 bis 14. Die Nut 60a kann beispielsweise an einem radial inneren Ende der der Intraokularlinse 11 abgewandten Seite des ersten Abschnitts 54 angeordnet sein, vergleiche Figur 9. Es ist auch denkbar, dass der Kartuschenkolben 10 zwei der Nuten 60a aufweist, die gegenüberliegend bezüglich des dritten Abschnitts 56 angeordnet sind.

Es ist auch denkbar, dass mehrere der Nuten 60b, 60d, 60e vorgesehen sind, die in unterschiedlichen Abständen zu dem dritten Abschnitt 56 angeordnet sind, vergleiche Figuren 10 bis 12. Figuren 10 bis 12 zeigen zudem, dass die Nuten 60b, 60d, 60e eine unterschiedliche Form haben können. Die Nuten 60b, 60d können im Querschnitt zumindest teilweise eckig sein. Die Nuten 60d, 60e können im Querschnitt zumindest teilweise rund sein. Es ist auch denkbar, dass in dem Kartuschenkolben die Nuten 60e im Querschnitt abwechselnd sowohl rund als auch eckig ausgeführt sein können.

Figuren 10 und 13 zeigen, dass der Kartuschenkolben 10 an seiner der Intraokularlinse 11 zugewandten Seite eine oder mehrere Vorderseitennuten 61 aufweisen kann. Dadurch wird eine Kontaktfläche der Intraokularlinse 11 an dem Kartuschenkolben 10 verkleinert, wodurch ein Festkleben der Intraokularlinse 11 an dem Kartuschenkolben 10 weniger wahrscheinlich wird. Die Vorderseitennuten 61a können beispielsweise im Querschnitt eckig sein, vergleiche Figur 10. Ebenso denkbar ist, dass die Vorderseitennuten 61b im Querschnitt rund sind, vergleiche Figur 13.

Die Fluidleitung 50 kann auch eine Gabelung 57 aufweisen, so dass die Fluidleitung 50 stromab der Gabelung 57 zwei Teilfluidleitungen 50a, 50b aufweist. Dadurch kann das Fluid 30 an zwei unterschiedlichen Öffnungen an dem ersten Kartuschenkolbenlängsende 51 austreten und somit gleichmäßiger in dem Kanal 6 stromab des Kartuschenkolbens 10 verteilt werden. Es ist auch denkbar, dass die Fluidleitung 50 mehrere der Gabelungen aufweist. Dadurch hat der Kanal 6 mindestens drei Teilfluidleitungen.

Die Kartusche 2 kann einen ersten Deckel aufweisen, der die erste Öffnung 7 abdichtet. Dadurch kann vermieden werden, dass die Intraokularlinse 11 während einer Lagerung der Kartusche 2 kontaminiert wird. In dem Fall, dass es sich bei der Intraokularlinse 11 um eine hydrophile Intraokularlinse handelt, die in einer Aufbewahrungsflüssigkeit zu lagern ist, kann durch den ersten Deckel und den Kartuschenkolben 10 vermieden werden, dass die Aufbewahrungsflüssigkeit aus dem Kanal 6 austritt. Zudem kann die Kartusche 2 einen zweiten Deckel aufweisen, der die zweite Öffnung 8 abdichtet. Um die Kartusche 2 mit der Spritze 3 zu kuppeln, ist vorher der zweite Deckel zu entfernen.

Figur 1 zeigt, dass die Intraokularlinse 11 einen Optikkörper 26 aufweisen kann, der vollständig zwischen der Verjüngung 9 und dem Kartuschenkolben 10 angeordnet ist. Zudem kann die Intraokularlinse 11 eine Haptik 27 aufweisen, die an dem Optikkörper 26 befestigt ist. Es ist denkbar, dass sich sowohl der Optikkörper 26 als auch die Haptik 27 in einem entspannten Zustand befinden.

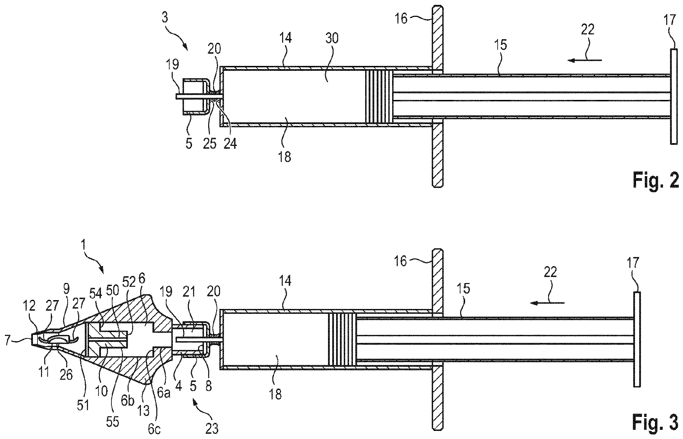

Wie es aus Figuren 1 und 3 ersichtlich ist, kann die Kartusche 2 eine Spitze 12 aufweisen, in der die erste Öffnung 7 angeordnet ist und die eingerichtet ist, in den Kapselsack eines Auges eingeführt zu werden. Die Spitze 12 kann in Richtung zu der ersten Öffnung 7 hin verjüngend ausgebildet sein. Zudem zeigen Figuren 1 und 3, dass der Kanal 6 gerade sein kann, was bedeutet, dass der Kanal 6 keine Kurve und/oder keine Umlenkung aufweist. Gemäß einer weiteren Ausführungsform kann der Kanal 6 jedoch eine Umlenkung aufweisen.

Zudem zeigen Figuren 1 und 3, dass die Kartusche 2 einen Kartuschenadapter 4 aufweisen kann, der eingerichtet ist, mit einem Spritzenadapter 5 der Spritze 3 in Eingriff zu stehen, um die Kartusche 2 mit der Spritze 3 zu kuppeln. Bei dem Kartuschenadapter 4 kann es sich beispielsweise um einen Steckeradapter einer Luer-Verbindung 23 handeln, wie es in Figuren 1 und 3 dargestellt ist. Alternativ ist denkbar, dass es sich bei dem Kartuschenadapter 4 um einen Buchsenadapter einer Luer-Verbindung 23 oder ein Teil eines Klemmverschlusses, eines Schnappverschlusses, eines Bajonettverschluss oder eines Schraubverschlusses handelt.

Figur 3 zeigt einen Injektor 1, der die Kartusche 2 und die Spritze 3 aufweist, wobei die Kartusche 2 mit der Spritze 3 gekuppelt ist. Die Spritze 3 weist das Fluid 30 auf und der Injektor 1 ist eingerichtet, durch ein Eindrücken des Fluids 30 in den Kanal 6 via die zweite Öffnung 8 mittels der Spritze 3 den Kartuschenkolben 10 in der Einschieberichtung 22 zu der ersten Öffnung 7 hin zu verschieben.

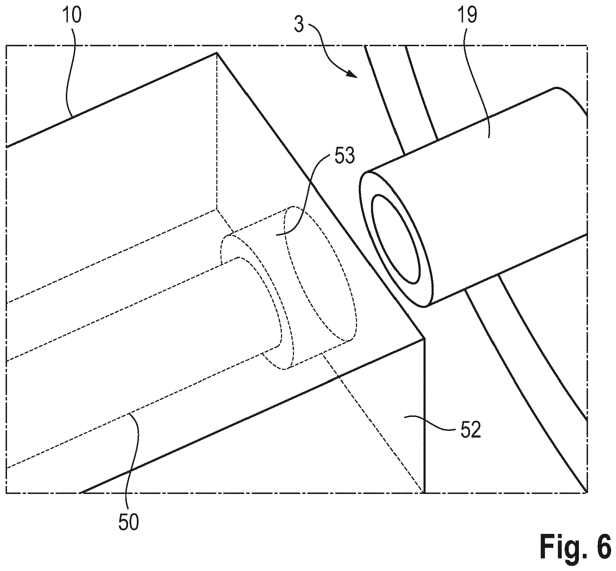

Aus Figuren 6 bis 16 ist ersichtlich, dass der Kartuschenkolben 10 an seinem der Spritze 3 zugewandten Ende eine Kartuschenkolbenaussparung 53 aufweisen kann, die eingerichtet ist, das dem Kartuschenkolben 10 zugewandte Längsende der Kanüle 19 aufzunehmen, wenn die Kartusche 2 mit der Spritze 3 gekuppelt wird. Die Kartuschenkolbenaussparung 53 kann dabei von einer Verbreiterung der Fluidleitung 50 gebildet sein, wobei die Verbreiterung im Bereich des zweiten Kartuschenkolbenlängsendes 52 angeordnet ist.

Figuren 2, 3 und 17 zeigen, dass die Spritze 3 eine Kanüle 19 aufweisen kann, durch die das Fluid 30 aus der Spritze 3 herausdrückbar ist. Die Kanüle 19 kann dabei so lang sein, dass sie in den Kanal 6 ragt. Zudem kann die Kanüle 19 starr ausgebildet sein. Die Spritze 3 kann einen Zylinder 14 und einen Spritzenkolben 15 aufweisen, der längsverschiebbar in dem Zylinder 4 gelagert ist, wobei der Zylinder 14 und der Spritzenkolben 15 einen Zylinderinnenraum 18 begrenzen, in dem das Fluid 30 angeordnet ist und der fluidleitend mit der Kanüle 19 verbunden ist.

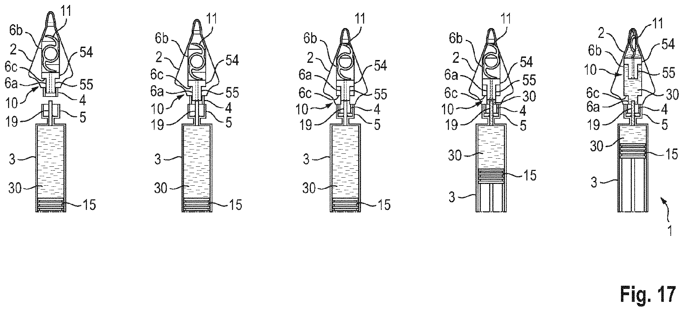

In Figur 17 ist der Injektor 1 zu fünf verschiedenen Zeitpunkten vor und nach seinem Zusammenbau sowie in seinem Betrieb dargestellt, wobei die Zeit von links nach rechts fortschreitet. Zu dem ersten Zeitpunkt sind die Kartusche 2 und die Spritze 3 voneinander getrennt. Zu dem zweiten Zeitpunkt ist der Anfang eines Kuppelns von der Kartusche 2 mit der Spritze 3 dargestellt, wobei der Kartuschenadapter 4 und der Spritzenadapter 5 sich berühren. Zu dem dritten Zeitpunkt sind die Kartusche 2 und die Spritze noch weiter angenähert und vollständig miteinander gekuppelt. Dies kann beispielsweise durch ein Verschrauben des Kartuschenadapters 4 mit dem Spritzenadapter 5 erfolgen. Dabei kann Kanüle 19 so lang sein, dass, wenn die Kartusche 2 mit der Spritze 3 gekuppelt wird, wie es zu dem dritten Zeitpunkt dargestellt ist, die Kanüle 19 den Kartuschenkolben 10 so weit in Richtung zu der ersten Öffnung 7 hin verschiebt, dass dabei die Intraokularlinse 11 vorgespannt wird. Es ist denkbar, dass die Intraokularlinse 11 dabei auch gefaltet wird. Indem die Intraokularlinse 11 vorgespannt und optional gefaltet wird, wird zwischen der Haptik 27 der Intraokularlinse 11 und der Wandung des Kanals 6 im Bereich der Verjüngung 9 eine Widerstandskraft R₁ erzeugt und es wird eine Abdichtung des Kanals 6 durch die Intraokularlinse 11 bewirkt. Wird nun bei dem Injektor 1 der Spritzenkolben 15 von einer ersten Position, vgl. Figur 2 und den dritten Zeitpunkt in Figur 17, entlang der Einschieberichtung 22 zu einer zweiten Position, vgl. Figur 3 und den vierten Zeitpunkt in Figur 17, verschoben, so verkleinert sich der Zylinderinnenraum 18 und das Fluid 30 wird von dem Zylinderinnenraum 18 in die Kanüle 19 gedrückt. Anschließend strömt das Fluid 30 aus der Kanüle 19 heraus und gelangt in den Kanal 6 in einem Bereich zwischen dem Kartuschenkolben 10 und der zweiten Öffnung 8. Von dort aus gelangt das Fluid 30 via die Fluidleitung 50 bis zu der Intraokularlinse 11. Dabei wird durch die Reibung des Fluids 30 in der Fluidleitung 50 eine Widerstandskraft R₂ erzeugt.

In Abhängigkeit von der Widerstandskraft R₂, durch Fluidreibung in der Fluidleitung 50 sowie einer Widerstandskraft R₃ in Form von Reibung zwischen dem Kartuschenkolben 10 und der Wand des Kanals 6 kommt es zu einer Beschleunigung des Kartuschenkolbens 10. Während dieser Phase ist die Widerstandskraft R₁ größer als die durch den Fluiddruck effektiv auf den Kartuschenkolben 10 wirkende Kraft F. Es gilt R₁>>F. Die wirkende Kraft reicht zunächst nur um die Intraokularlinse 11 vorzuspannen. Gleichzeitig wird der Kartuschenkolben 10 aufgrund einer von dem Fluid 30 auf das zweite Kartuschenkolbenlängsende 52 ausgeübten Kraft F in Richtung zu der ersten Öffnung 7 hin verschoben. Durch die Reibung des Kartuschenkolbens 10 an der Wand des Kanals 6 wird die Widerstandskraft R₃ erzeugt. In dieser Phase gilt (R₂ > R₃) < R₁. Durch einen Sog und die resultierende Verschiebung des Kartuschenkolbens 10 in Folge der Fluidreibung R₂ vergrößert sich die Angriffsfläche des Fluids 30 auf der Seite des zweiten Kartuschenkolbenlängsendes 52. Durch weiteres Drücken des Spritzenkolbens 15 steigt der Fluiddruck im Kanal 6 zusätzlich an, wodurch es zu einem zusätzlichen Anstieg der auf den Kartuschenkolben 10 wirkenden Kraft kommt, sodass der Kartuschenkolben 10 in Richtung zur ersten Öffnung hin verschoben wird und dabei die Intraokularlinse 11 gefaltet wird

Zu dem vierten Zeitpunkt ist das zweite Kartuschenkolbenlängsende 52 in dem ersten Kanalabschnitt 6a angeordnet und das Fluid 30 greift auch an dem zweiten Kartuschenkolbenlängsende 52 an. Zu dem fünften Zeitpunkt ist das zweite Kartuschenkolbenlängsende 52 in dem zweiten Kanalabschnitt 6b angeordnet und das Fluid 30 greift zusätzlich an die der Intraokularlinse 11 abgewandte Seite des ersten Abschnitts 54 an. Unter der Annahme, dass zu dem vierten Zeitpunkt und dem fünften Zeitpunkt der Druck des Fluids 30 gleich hoch ist, ist die Kraft F zu dem fünften Zeitpunkt stärker als zu dem vierten Zeitpunkt, so dass gilt: F>R₁. Damit ist es nun möglich, die Intraokularlinse 11 aus der ersten Öffnung 7 heraus zu drücken.

Wie es aus Figur 3 ersichtlich ist, kann die Kartusche 2 einen Kartuschenadapter 4 aufweisen und die Spritze 3 kann einen Spritzenadapter 5 aufweisen, durch den die Kanüle 19 sich erstreckt, wobei der Kartuschenadapter 4 und der Spritzenadapter 5 miteinander in Eingriff stehen, wodurch die Kartusche 2 mit der Spritze 3 gekuppelt ist und der Injektor 1 im Bereich des Kartuschenadapters 4 und des Spritzenadapters 5 abgedichtet ist. Um den Kartuschenadapter 4 mit dem Spritzenadapter 5 in Eingriff zu bringen, kann dies mittels einer Bewegung der Kartusche 2 relativ zu der Spritze 3 in Richtung entgegen der Strömungsrichtung erfolgen, die das Fluid 30 in der Kanüle 19 annimmt, wenn sie aus der Spritze 3 herausgedrückt wird, bzw. entgegen der Einschieberichtung 22 erfolgen. Der Kartuschenadapter 4 und der Spritzenadapter 5 können eingerichtet sein, formschlüssig miteinander in Eingriff zu stehen, wenn die Kartusche 2 mit der Spritze 3 gekuppelt ist. Figur 3 zeigt, dass der Kartuschenadapter 4 und der Spritzenadapter 5 eine Luer-Verbindung 23 bilden können. Bei der Luer-Verbindung 23 wird der Injektor 1 an denjenigen Flächen des Kartuschenadapters 4 und des Spritzenadapters 5 abgedichtet, an denen der Kartuschenadapter 4 und der Spritzenadapter 5 sich berühren. Bei der Luer-Verbindung 23 ist denkbar, dass der Kartuschenadapter 4 der Steckeradapter ist und der Spritzenadapter 5 der Buchsenadapter ist, wie es auch in Figur 3 dargestellt ist. Alternativ ist denkbar, dass der Kartuschenadapter 4 der Buchsenadapter ist und der Spritzenadapter 5 der Steckeradapter ist. Alternativ zu der Luer-Verbindung 23 können der Kartuschenadapter 4 und der Spritzenadapter 5 einen Klemmverschluss, einen Schnappverschluss, einen Bajonettverschluss oder einen Schraubverschluss bilden.

Der Injektor 1 kann einen Adapterinnenraum 21 aufweisen, der unmittelbar an der zweiten Öffnung 8 angrenzt und von dem Kartuschenadapter 4 begrenzt ist. Zudem kann die Spritze 3 ein Rohr 20 aufweisen, das ein erstes Längsende 24 und ein zweites Längsende 25 aufweist. Das erste Längsende 24 ist an dem Zylinder 14 befestigt und der Spritzenadapter 5 ist an dem zweiten Längsende 25 befestigt. Es ist denkbar, dass sich die Kanüle 19 durch das Rohr 21 erstreckt. Dadurch kann die Kanüle 19 besonders fest an dem Zylinder 14 befestigt werden.

Figuren 1 und 3 zeigen, dass die Kartusche 2 einen Kartuschenflügel 13 aufweisen kann, der von der verbliebenen Kartusche 2 nach außen absteht. Dadurch kann die Kartusche 2 gut angefasst werden und somit einfach von Hand mit der Spritze 3 gekuppelt werden. Figuren 2 und 3 zeigen, dass der Zylinder 14 an seinem der Kanüle 19 abgewandten Ende einen Griff 16 aufweisen kann, der von dem verbliebenen Zylinder 14 nach außen absteht. Zudem kann der Spritzenkolben 15 an seinem der Kanüle 19 abgewandten Ende eine Verdickung 17 aufweisen, die von dem verbliebenen Spritzenkolben 15 nach außen absteht.

Das Fluid kann ein Gleitmittel und/oder eine physiologische Salzlösung aufweisen oder aus dem Gleitmittel und/oder der physiologischen Salzlösung bestehen. Bei dem Gleitmittel kann es sich um ein sogenanntes opthalmisches Viskoelastikum handeln (engl.: opthalmic viscoelastic device (OVD)). Das opthalmische Viskoelastikum hat eine vergleichsweise hohe Viskosität und eignet sich daher besonders, um den Kartuschenkolben 10 anzutreiben.

### Bezugszeichenliste

1 Injektor
2 Kartusche
3 Spritze
4 Kartuschenadapter
5 Spritzenadapter
6 Kanal
6a erster Kanalabschnitt
6b zweiter Kanalabschnitt
6c Stufe
7 erste Öffnung
8 zweite Öffnung
9 Verjüngung
10 Kartuschenkolben
11 Intraokuarlinse
12 Spitze
13 Kartuschenflügel
14 Zylinder
15 Spritzenkolben
16 Griff
17 Verdickung
18 Zylinderinnenraum
19 Kanüle
20 Rohr
21 Adapterinnenraum
22 Einschieberichtung
23 Luer-Verbindung
24 erstes Längsende
25 zweites Längsende
26 Optikkörper
27 Haptik
30 Fluid
50 Fluidleitung
50a Teilfluidleitung
50b Teilfluidleitung
51 erstes Kartuschenkolbenlängsende
52 zweites Kartuschenkolbenlängsende
53 Kartuschenkolbenaussparung
54 erster Abschnitt
55 zweiter Abschnitt
56 dritter Abschnitt
57 Gabelung
58a Aussparung
58b weitere Aussparung
60 Nut
61 Vorderseitennut
62 Querseitennut

## Patentansprüche

1. Kartusche mit einer ersten Öffnung (7), einer zweiten Öffnung (8), einem Kanal (6), der sich von der ersten Öffnung (7) bis zu der zweiten Öffnung (8) erstreckt und eine Verjüngung (9) aufweist, einer Intraokularlinse (11), die in dem Kanal (6) zwischen der ersten Öffnung (7) und der zweiten Öffnung (8) angeordnet ist, und einem Kartuschenkolben (10), der in dem Kanal (6) zwischen der Intraokularlinse (11) und der zweiten Öffnung (8) angeordnet ist und eingerichtet ist, durch ein Verschieben des Kartuschenkolbens (10) zu der ersten Öffnung (7) hin die Intraokularlinse (11) zuerst vorzuspannen, danach durch ein Verschieben der Intraokularlinse (11) in der Verjüngung (9) zu falten und anschließend via die erste Öffnung (7) aus dem Kanal (6) zu drücken, wobei die Kartusche (2) eingerichtet ist, mit einer Spritze (3) gekuppelt zu werden, so dass mittels der Spritze (3) via die zweite Öffnung (8) ein Fluid (30) in den Kanal (6) eindrückbar ist und dadurch der Kartuschenkolben (10) in einer Einschieberichtung (22) hin zu der ersten Öffnung (7) antreibbar ist, wobei der Kartuschenkolben (10) ein erstes Kartuschenkolbenlängsende (51) und ein zweites Kartuschenkolbenlängsende (52) aufweist, **dadurch gekennzeichnet, dass** der Kartuschenkolben (10) eine Fluidleitung (50) aufweist, die sich von dem ersten Kartuschenkolbenlängsende (51) bis zu dem zweiten Kartuschenkolbenlängsende (52) erstreckt und via die das Fluid (30) von dem zweiten Kartuschenkolbenlängsende (52) bis zu dem ersten Kartuschenkolbenlängsende (51) und anschließend bis zu der Intraokularlinse (11) eindrückbar ist.

2. Kartusche gemäß Anspruch 1, wobei das erste Kartuschenkolbenlängsende (51) projiziert in der Einschieberichtung (22) einen größeren Querschnitt hat als das zweite Kartuschenkolbenlängsende (52) projiziert in der Einschieberichtung (22).

3. Kartusche gemäß Anspruch 2, wobei der Kanal (6) einen ersten Kanalabschnitt (6a), in dem das zweite Kartuschenkolbenlängsende (52) angeordnet ist, bevor das Fluid (30) in den Kanal (6) eingedrückt ist, und der einen ersten Querschnitt aufweist, dessen Normale parallel zu der Einschieberichtung (22) angeordnet ist und im Wesentlichen dem in der Einschieberichtung (22) projizierten zweiten Kartuschenkolbenlängsende (2) entspricht, und einen zweiten Kanalabschnitt (6b) aufweist, der zwischen dem ersten Kanalabschnitt (6a) und der ersten Öffnung (7) angeordnet ist und einen zweiten Querschnitt aufweist, dessen Normale parallel zu der Einschieberichtung (22) angeordnet ist und der größer als der erste Querschnitt ist, wobei der Abstand von der ersten Öffnung (7) zu dem ersten Kanalabschnitt (6a) länger ist als die Erstreckung des Kartuschenkolbens (10) in der Einschieberichtung (22).

4. Kartusche gemäß einem der Ansprüche 1 bis 3, wobei der Kartuschenkolben (10) einen ersten Abschnitt (54), der das erste Kartuschenkolbenlängsende (51) aufweist und flexibel ist, einen zweiten Abschnitt (55), der das zweite Kartuschenkolbenlängsende (52) aufweist, und einen dritten Abschnitt (56) aufweist, der zwischen dem ersten Abschnitt (54) und dem zweiten Abschnitt (55) angeordnet ist und eine Aussparung (58a) aufweist, die eingerichtet ist, Teile des ersten Abschnitts (54) aufzunehmen, wenn der Kartuschenkolben (10) in die Verjüngung (9) gelangt.

5. Kartusche gemäß Anspruch 4, wobei der erste Abschnitt (54) an seiner der Intraokularlinse (11) abgewandten Seite eine Nut (60) aufweist.

6. Kartusche gemäß einem der Ansprüche 1 bis 5, wobei die Fluidleitung (50) eine Kurve aufweist.

7. Injektor mit einer Kartusche (2) gemäß einem der Ansprüche 1 bis 6 und der Spritze (3), wobei die Kartusche (2) mit der Spritze (3) gekuppelt ist, die Spritze (3) das Fluid (30) aufweist und der Injektor (1) eingerichtet ist, durch ein Eindrücken des Fluids (30) in den Kanal (6) via die zweite Öffnung (8) mittels der Spritze (3) den Kartuschenkolben (10) in der Einschieberichtung (22) zu der ersten Öffnung (7) hin zu verschieben.

8. Injektor gemäß Anspruch 7, wobei die Spritze (3) eine Kanüle (19) aufweist, durch die das Fluid (30) aus der Spritze (3) herausdrückbar ist.

9. Injektor gemäß Anspruch 8, wobei die Kanüle (19) so lang ist, dass, wenn die Kartusche (2) mit der Spritze (3) gekuppelt wird, die Kanüle (19) den Kartuschenkolben (10) so weit in Richtung zu der ersten Öffnung (7) hin verschiebt, dass dabei die Intraokularlinse (11) vorgespannt wird.

10. Injektor gemäß Anspruch 8 oder 9, wobei der Kartuschenkolben (10) an seinem der Spritze (3) zugewandten Ende eine Kartuschenkolbenaussparung (53) aufweist, die eingerichtet ist, das dem Kartuschenkolben (10) zugewandte Längsende der Kanüle (19) aufzunehmen, wenn die Kartusche (2) mit der Spritze (3) gekuppelt wird.

## Claims

1. Cartridge with a first opening (7), a second opening (8), a channel (6), which extends from the first opening (7) to the second opening (8) and has a taper (9), an intraocular lens (11), which is arranged in the channel (6) between the first opening (7) and the second opening (8), and a cartridge plunger (10), which is arranged in the channel (6) between the intraocular lens (11) and the second opening (8) and is set up to first preload the intraocular lens (11) by displacing the cartridge plunger (10) toward the first opening (7), then to fold the intraocular lens (11) by displacing it in the taper (9) and then to push the lens out of the channel (6) via the first opening (7), wherein the cartridge (2) is set up to be coupled with a syringe (3), so that a fluid (30) can be forced into the channel (6) via the second opening (8) by means of the syringe (3) and the cartridge plunger (10) can thereby be propelled in a direction of insertion (22) toward the first opening (7), wherein the cartridge plunger (10) has a first cartridge-plunger longitudinal end (51) and a second cartridge-plunger longitudinal end (52), **characterized in that** the cartridge plunger (10) has a fluid line (50), which extends from the first cartridge-plunger longitudinal end (51) to the second cartridge-plunger longitudinal end (52) and via which the fluid (30) can be forced in from the second cartridge-plunger longitudinal end (52) to the first cartridge-plunger longitudinal end (51) and then to the intraocular lens (11).

2. Cartridge according to Claim 1, wherein the first cartridge-plunger longitudinal end (51), projected in the direction of insertion (22), has a larger cross section than the second cartridge-plunger longitudinal end (52), projected in the direction of insertion (22).

3. Cartridge according to Claim 2, wherein the channel (6) has a first channel portion (6a), in which the second cartridge-plunger longitudinal end (52) is arranged before the fluid (30) is forced into the channel (6) and which has a first cross section, the normal of which is arranged parallel to the direction of insertion (22) and essentially corresponds to the second cartridge-plunger longitudinal end (2) projected in the direction of insertion (22), and a second channel portion (6b), which is arranged between the first channel portion (6a) and the first opening (7) and has a second cross section, the normal of which is arranged parallel to the direction of insertion (22) and which is larger than the first cross section, wherein the distance from the first opening (7) to the first channel portion (6a) is longer than the extent of the cartridge plunger (10) in the direction of insertion (22).

4. Cartridge according to one of Claims 1 to 3, wherein the cartridge plunger (10) has a first portion (54), which has the first cartridge-plunger longitudinal end (51) and is flexible, a second portion (55), which has the second cartridge-plunger longitudinal end (52), and a third portion (56), which is arranged between the first portion (54) and the second portion (55) and has a recess (58a), which is set up to receive parts of the first portion (54) when the cartridge plunger (10) enters the taper (9).

5. Cartridge according to Claim 4, wherein the first portion (54) has a groove (60) on its side facing away from the intraocular lens (11).

6. Cartridge according to one of Claims 1 to 5, wherein the fluid line (50) has a curve.

7. Injector with a cartridge (2) according to one of Claims 1 to 6 and the syringe (3), wherein the cartridge (2) is coupled to the syringe (3), the syringe (3) contains the fluid (30) and the injector (1) is set up to displace the cartridge plunger (10) in the direction of insertion (22) toward the first opening (7) by forcing the fluid (30) into the channel (6) via the second opening (8) by means of the syringe (3).

8. Injector according to Claim 7, wherein the syringe (3) has a cannula (19) through which the fluid (30) can be forced out of the syringe (3).

9. Injector according to Claim 8, wherein the cannula (19) is of such a length that, when the cartridge (2) is coupled to the syringe (3), the cannula (19) displaces the cartridge plunger (10) so far in the direction of the first opening (7) that the intraocular lens (11) is thereby preloaded.

10. Injector according to Claim 8 or 9, wherein the cartridge plunger (10) has at its end facing the syringe (3) a cartridge-plunger recess (53), which is set up to receive the longitudinal end of the cannula (19) facing the cartridge plunger (10) when the cartridge (2) is coupled to the syringe (3).

## Revendications

1. Cartouche avec une première ouverture (7), une deuxième ouverture (8), un canal (6) qui s'étend de la première ouverture (7) à la deuxième ouverture (8) et qui présente un rétrécissement (9), une lentille intraoculaire (11) qui est agencée dans le canal (6) entre la première ouverture (7) et la deuxième ouverture (8), et un piston de cartouche (10), qui est agencé dans le canal (6) entre la lentille intraoculaire (11) et la deuxième ouverture (8) et qui est adapté pour d'abord précontraindre la lentille intraoculaire (11) par un déplacement du piston de cartouche (10) vers la première ouverture (7), puis pour la plier par un déplacement de la lentille intraoculaire (11) dans le rétrécissement (9) et ensuite pour la pousser hors du canal (6) via la première ouverture (7), la cartouche (2) étant adaptée pour être couplée à une seringue (3), de telle sorte qu'un fluide (30) peut être injecté dans le canal (6) au moyen de la seringue (3) via la deuxième ouverture (8) et le piston de cartouche (10) peut ainsi être entraîné dans une direction d'insertion (22) vers la première ouverture (7), le piston de cartouche (10) présentant une première extrémité longitudinale de piston de cartouche (51) et une deuxième extrémité longitudinale de piston de cartouche (52), **caractérisée en ce que** le piston de cartouche (10) présente un conduit de fluide (50) qui s'étend de la première extrémité longitudinale de piston de cartouche (51) jusqu'à la deuxième extrémité longitudinale de piston de cartouche (52) et via lequel le fluide (30) peut être injecté de la deuxième extrémité longitudinale de piston de cartouche (52) jusqu'à la première extrémité longitudinale de piston de cartouche (51) et ensuite jusqu'à la lentille intraoculaire (11).

2. Cartouche selon la revendication 1, dans laquelle la première extrémité longitudinale de piston de cartouche (51) projetée dans la direction d'insertion (22) a une section transversale plus grande que la deuxième extrémité longitudinale de piston de cartouche (52) projetée dans la direction d'insertion (22).

3. Cartouche selon la revendication 2, dans laquelle le canal (6) présente une première section de canal (6a) dans laquelle la deuxième extrémité longitudinale de piston de cartouche (52) est agencée avant que le fluide (30) ne soit injecté dans le canal (6), et qui présente une première section transversale dont la normale est agencée parallèlement à la direction d'insertion (22) et correspond essentiellement à la deuxième extrémité longitudinale de piston de cartouche (2) projetée dans la direction d'insertion (22), et une deuxième section de canal (6b) qui est agencée entre la première section de canal (6a) et la première ouverture (7) et qui présente une deuxième section transversale dont la normale est agencée parallèlement à la direction d'insertion (22) et qui est plus grande que la première section transversale, la distance entre la première ouverture (7) et la première section de canal (6a) étant plus longue que l'extension du piston de cartouche (10) dans la direction d'insertion (22).

4. Cartouche selon l'une quelconque des revendications 1 à 3, dans laquelle le piston de cartouche (10) présente une première section (54) présentant la première extrémité longitudinale de piston de cartouche (51) et étant flexible, une deuxième section (55) présentant la deuxième extrémité longitudinale de piston de cartouche (52), et une troisième section (56) agencée entre la première section (54) et la deuxième section (55) et présentant un évidement (58a) adapté pour recevoir des parties de la première section (54) lorsque le piston de cartouche (10) entre dans le rétrécissement (9).

5. Cartouche selon la revendication 4, dans laquelle la première section (54) présente une rainure (60) sur son côté détourné de la lentille intraoculaire (11).

6. Cartouche selon l'une quelconque des revendications 1 à 5, dans laquelle le conduit de fluide (50) présente une courbe.

7. Injecteur avec une cartouche (2) selon l'une quelconque des revendications 1 à 6 et la seringue (3), la cartouche (2) étant couplée à la seringue (3), la seringue (3) présentant le fluide (30) et l'injecteur (1) étant adapté pour déplacer le piston de cartouche (10) dans la direction d'insertion (22) vers la première ouverture (7) par une injection du fluide (30) dans le canal (6) via la deuxième ouverture (8) au moyen de la seringue (3).

8. Injecteur selon la revendication 7, dans lequel la seringue (3) présente une canule (19) par laquelle le fluide (30) peut être expulsé de la seringue (3).

9. Injecteur selon la revendication 8, dans lequel la canule (19) est d'une longueur telle que, lorsque la cartouche (2) est couplée à la seringue (3), la canule (19) déplace le piston de cartouche (10) en direction de la première ouverture (7) dans une mesure telle que la lentille intraoculaire (11) est alors précontrainte.

10. Injecteur selon la revendication 8 ou 9, dans lequel le piston de cartouche (10) présente, à son extrémité tournée vers la seringue (3), un évidement de piston de cartouche (53) qui est adapté pour recevoir l'extrémité longitudinale de la canule (19) tournée vers le piston de cartouche (10) lorsque la cartouche (2) est couplée à la seringue (3).
